# EUROPEAN PATENT APPLICATION

(11) **EP 3 878 354 A1**
(43) Date of publication of application: **15.09.2021**
(21) Application number: 21161208.0
(22) Date of filing: 08.03.2021
(51) Int. Cl.: A61B 5/00, A61B 5/339, A61B 5/367, A61B 18/14, G06F 3/0482

(54) **ELECTROPHYSIOLOGICAL USER INTERFACE**

(30) Priority: 09.03.2020 US 202062986856 P; 05.01.2021 US 202117141754
(71) Applicant: Biosense Webster (Israel) Ltd, Yokneam, 2066717 (IL)
(72) Inventor: KATZ, Natan Sharon, 2066717 Yokneam (IL); COHEN, Benjamin, 2066717 Yokneam (IL); DVORKIN, Vladimir, 2066717 Yokneam (IL); IDOV, Roman, 2066717 Yokneam (IL)
(74) Representative: Carpmaels & Ransford LLP

(57) **Abstract**

In one embodiment, a medical mapping method includes receiving position data from a positioning sub-system configured to sense a position of a probe inserted into a body cavity of a living subject, computing position coordinates of a distal end of the probe, rendering to a display a user interface screen including a display pane including an anatomical map of the body cavity responsively to the computed position coordinates, and a tab-based menu above the pane, receiving a user selection selecting a tab of the tab-based menu, and showing a horizontal ribbon of the selected tab above the pane in the user interface screen responsively to the received user selection.

## Description

### RELATED APPLICATION INFORMATION

The present application claims benefit of US Provisional Patent Application S/N 62/986,856 to Katz, et al., filed 9 March 2020, the disclosure of which is hereby incorporated herein by reference.

### FIELD OF THE INVENTION

The present invention relates to medical systems, and in particular, but not exclusively, to user interfaces for medical applications.

### COPYRIGHT NOTICE

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all rights whatsoever.

### BACKGROUND

Body parts are mapped to provide information to a physician. By way of example, cardiac mapping is performed to visualize various features of different parts of the heart, including indicating surfaces of tissue, electrical features such as local activation times (LATs) using color maps and/or arrows, and medical treatment features using tags, such as VisiTags of Biosense Webster®, Inc. of Irvine, California, United States. A VisiTag indicates a location of an ablation performed by a catheter. The VisiTag may also include additional information such as ablation time used to create the ablation. VisiTag location information may be useful for making future ablation decisions, by way of example only. Medical instruments may also be visualized along with the cardiac map.

### SUMMARY

There is provided in accordance with an embodiment of the present disclosure, a medical mapping method including receiving position data from a positioning sub-system configured to sense a position of a probe inserted into a body cavity of a living subject, computing position coordinates of a distal end of the probe responsively to the position data, rendering to a display a user interface screen including a display pane including an anatomical map of the body cavity responsively to the computed position coordinates, and a tab-based menu above the pane, receiving a user selection selecting a tab of the tab-based menu, and showing a horizontal ribbon of the selected tab above the pane in the user interface screen responsively to the received user selection.

Further in accordance with an embodiment of the present disclosure the display pane is not resized or partially hidden responsively to the showing of the horizontal ribbon.

Still further in accordance with an embodiment of the present disclosure, the method includes receiving a user selection of a selectable control of the ribbon, and showing a contextual tab in the user interface screen responsively to the user selection of the selectable control, the contextual tab including other selectable controls.

Additionally, in accordance with an embodiment of the present disclosure the other selectable controls of the contextual tab include controls to create a new map or a new anatomical structure from an existing map.

Moreover, in accordance with an embodiment of the present disclosure, the method includes receiving a user selection of a point on the anatomical map, and showing a contextual tab in the user interface screen responsively to the user selection of the point, the contextual tab including data about the selected point.

Further in accordance with an embodiment of the present disclosure the ribbon includes at least one data readout element about any one or more of the following ablation data, a tissue property, a selected point of the anatomical map, and tool connectivity.

Still further in accordance with an embodiment of the present disclosure the ribbon includes at least one control to perform any one or more of the following: creation of the anatomical map, formatting the anatomical map, creation of an anatomical structure, editing the anatomical map, configuring the probe, taking a screen snapshot of the pane, recording the pane, selecting a preset layout of which panes to show, and creating custom layouts.

Additionally, in accordance with an embodiment of the present disclosure, the method includes receiving a user selection of a selectable control of the ribbon to add the selectable control to a quick access toolbar, and showing a miniaturized representation of the selectable control in the quick access toolbar.

Moreover, in accordance with an embodiment of the present disclosure, the method includes receiving at least one signal from at least one sensor of the probe, and rendering the anatomical map responsively to the at least one signal.

Further in accordance with an embodiment of the present disclosure, the method includes sensing electrical signals of the body cavity with at least one electrode of the probe, wherein the rendering the anatomical map includes rendering the anatomical map with electrophysiological data indicators responsively to the sensed electrical signals.

Still further in accordance with an embodiment of the present disclosure, the method includes rendering a representation of the probe in the display pane responsively to the computed position coordinates.

Additionally, in accordance with an embodiment of the present disclosure, the method includes rendering ablation indicators on the anatomical map.

There is also provided in accordance with another embodiment of the present disclosure, a medical system, including a probe including a distal end, and configured to be inserted into a body cavity of a living subject, a positioning sub-system configured to sense a position of the probe, a display, and a processor configured to receive position data from the positioning sub-system, compute position coordinates of the distal end of the probe responsively to the position data, render to the display a user interface screen including a display pane including an anatomical map of the body cavity responsively to the computed position coordinates, and a tab-based menu above the pane, receive a user selection selecting a tab of the tab-based menu, and show a ribbon of the selected tab in the user interface screen responsively to the received user selection.

Moreover, in accordance with an embodiment of the present disclosure the display pane is not resized or partially hidden responsively to the showing of the horizontal ribbon.

Further in accordance with an embodiment of the present disclosure the processor is configured to receive a user selection of a selectable control of the ribbon, and show a contextual tab in the user interface screen responsively to the user selection of the selectable control, the contextual tab including other selectable controls.

Still further in accordance with an embodiment of the present disclosure the other selectable controls of the contextual tab include controls to create a new map or a new anatomical structure from an existing map.

Additionally, in accordance with an embodiment of the present disclosure the processor is configured to receive a user selection of a point on the anatomical map, and show a contextual tab in the user interface screen responsively to the user selection of the point, the contextual tab including data about the selected point.

Moreover, in accordance with an embodiment of the present disclosure the ribbon includes at least one data readout element about any one or more of the following ablation data, a tissue property, a selected point of the anatomical map, and tool connectivity.

Further in accordance with an embodiment of the present disclosure the ribbon includes at least one control to perform any one or more of the following: creation of the anatomical map, formatting the anatomical map, creation of an anatomical structure, editing the anatomical map, configuring the probe, taking a screen snapshot of the pane, recording the pane, selecting a preset layout of which panes to show, and creating custom layouts.

Still further in accordance with an embodiment of the present disclosure the processor is configured to receive a user selection of a selectable control of the ribbon to add the selectable control to a quick access toolbar, and show a miniaturized representation of the selectable control in the quick access toolbar.

Additionally, in accordance with an embodiment of the present disclosure the probe includes at least one sensor, and the processor is configured to receive at least one signal from the at least one sensor of the probe, and render the anatomical map responsively to the at least one signal.

Moreover, in accordance with an embodiment of the present disclosure the probe includes at least one electrode, which is configured to sense electrical signals of the body cavity, and the processor is configured to render the anatomical map with electrophysiological data indicators responsively to the sensed electrical signals.

Further in accordance with an embodiment of the present disclosure the processor is configured to render a representation of the probe in the display pane responsively to the computed position coordinates.

Still further in accordance with an embodiment of the present disclosure the processor is configured to render ablation indicators on the anatomical map.

There is also provided in accordance with still another embodiment of the present disclosure, a software product, including a non-transient computer-readable medium in which program instructions are stored, which instructions, when read by a central processing unit (CPU), cause the CPU to receive position data from a positioning sub-system configured to sense a position of a probe inserted into a body cavity of a living subject, compute position coordinates of a distal end of the probe responsively to the position data, render to a display a user interface screen including a display pane including an anatomical map of the body cavity responsively to the computed position coordinates, and a tab-based menu above the pane, receive a user selection selecting a tab of the tab-based menu, and show a ribbon of the selected tab in the user interface screen responsively to the received user selection.

### BRIEF DESCRIPTION OF THE DRAWINGS

The present invention will be understood from the following detailed description, taken in conjunction with the drawings in which:
Fig. 1 is a partly pictorial, partly block diagram view of a mapping system constructed and operative in accordance with an exemplary embodiment of the present invention;
Figs. 2A-M are views of a user interface screen constructed and operative in accordance with an exemplary embodiment of the present invention; and
Figs. 3A-B are flowcharts including steps in a method of operation of the system of Fig. 1.

### DESCRIPTION OF EXAMPLE EMBODIMENTS

### OVERVIEW

As previously mentioned, mapping provides information to a physician regarding the state of a body part (e.g., the heart) in order for the physician to develop a treatment plan for the body part. The physician may also review other data, for example, electrocardiograms, previous ablation data, and local activation time (LAT) data superimposed on a map of the body part. The mapping may be performed using a catheter inserted into the body part with a view to performing treatment, such as ablation, while the catheter is still in the body part. In other cases, the catheter used for mapping may be removed before treatment is performed.

Analyzing the mapping data correctly, and sometimes quickly, is essential for correct and timely treatment. A user interface used to display the mapping data may offer various options for creating, editing and displaying the map, including different views, different formatting schemes, different coloring schemes, and adding annotations among other functions. The user interface may also provide options for displaying graphs and charts to convey the medical data.

It is important that the user interface is intuitive to use and allows the physician to create new maps and edit and format existing maps and other data while limiting blocking the view of the map(s).

Embodiments of the present invention solve the above problems by providing a medical mapping system including rendering a user interface including a display pane, which includes an anatomical map and other medical data, with a tab-based menu above the pane. The tab-based menu includes selectable ribbons including data readout elements and selectable controls (such as action buttons, sliders, toggle buttons, drop-down buttons, etc.) so that the menu may be navigated while limiting blocking of the display pane, which includes the anatomical map and other medical data. The tab-based menu also provides ease of use as the physician knows that most of the actions and readout elements are located in the menu ribbons.

In some embodiments, a medical mapping method includes receiving position data from a positioning sub-system which senses a position of a probe inserted into a body cavity of a living subject, and receiving at least one signal from at least one sensor of the probe. The sensor may provide position signals or other signals which may be used to compute force, proximity, tissue impedance, tissue temperature or electrical activity of the tissue by sensing electrical signals of the body cavity with at least one electrode of the probe. The method may also include computing position coordinates of a distal end of the probe responsively to the position data and rendering, to a display, a user interface screen including: a display pane including an anatomical map of the body cavity responsively to the computed position coordinates; and a tab-based menu above the pane. In some embodiments, the method may include rendering the anatomical map responsively to received signal(s) (e.g., position signal(s) and/or electrical signals captured from the tissue of the body cavity. In some embodiments, rendering the anatomical map may include rendering the anatomical map with electrophysiological data indicators, such as a LAT map using coloring, shading and/or vectors, responsively to the sensed electrical signals. In some embodiments, the method may include rendering a representation of the probe in the display pane responsively to the computed position coordinates and/or rendering ablation indicators (e.g., VisiTags) on the anatomical map.

The method may also include receiving a user selection selecting a tab of the tab-based menu and showing a horizontal ribbon of the selected tab above the pane in the user interface screen responsively to the received user selection. Generally, the display pane is not resized or even partially hidden responsively to showing the horizontal ribbon in the menu, thereby keeping the display pane clear for effective use by the physician. The method may include receiving a user selection of a selectable control of the displayed ribbon and performing an action associated with the selected control.

The ribbons of the menu may include any suitable data or controls. In some embodiments, the ribbons may include one or more data readout elements about any one or more of the following: ablation data; a tissue property; a selected point of the anatomical map; and tool connectivity. Additionally, or alternatively, the ribbons may include one or more controls to perform any one or more of the following: creation of the anatomical map; formatting the anatomical map; creation of an anatomical structure; editing the anatomical map; configuring the probe, taking a screen snapshot of the pane; recording the pane; selecting a preset layout of which panes to show; and creating custom layouts.

The user interface may include contextual activated tabs. For example, the method may include receiving a user selection of a selectable control of one of the ribbons and showing a contextual tab in the user interface screen responsively to the user selection of the selectable control. The contextual tab may include selectable controls, for example, to create a new map or a new anatomical structure from an existing map. One example of a contextual tab is a point-activated contextual tab. For example, the method may include receiving a user selection of a point on the anatomical map and showing a contextual tab (including data about the selected point) in the user interface screen responsively to the user selection of the point.

To allow ease-of-access to commonly used functions, the user interface may include a customizable quick access toolbar to which commonly used functions may be added. For example, the method may include receiving a user selection of a selectable control of one of the ribbons to add the selectable control to a quick access toolbar and showing a miniaturized representation of the selectable control in the quick access toolbar. The quick access toolbar may be shown above or below the active ribbon.

### SYSTEM DESCRIPTION

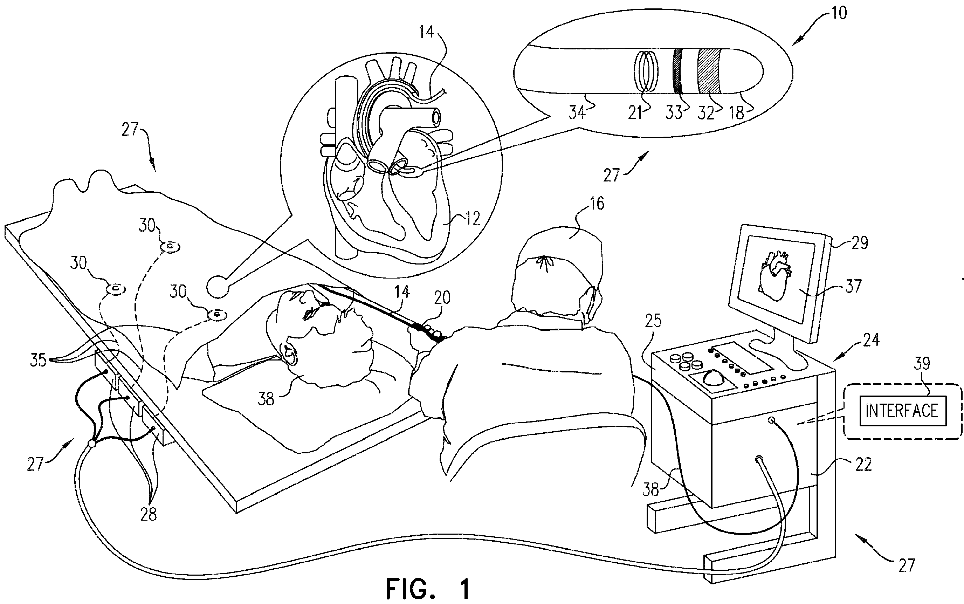

Turning now to the drawings, reference is initially made to Fig. 1, which is a pictorial illustration of a mapping system 10, constructed and operative in accordance with a disclosed embodiment of the invention, for evaluating electrical activity and optionally for performing ablative procedures on a heart 12 (or any other suitable body cavity) of a living subject 38. The system comprises a probe 14, such as a catheter, which is percutaneously inserted by an operator 16 through the patient's vascular system into a chamber or vascular structure of the heart 12. The operator 16, who is typically a physician, brings a distal tip 18 of the probe 14 into contact with the heart wall, for example, at an ablation target site or to capture electrical potentials over time at multiple sample location over a surface of one or more chambers of the heart 12. Electrical activation maps may be prepared, according to the methods disclosed in U.S. Patents 6,226,542, 6,301,496, and 6,892,091. One commercial product embodying elements of the system 10 is available as the CARTO® 3 System, available from Biosense Webster, Inc., 33 Technology Drive, Irvine, CA 92618 USA. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the probe to one or more electrodes at the distal tip 18, which apply the radiofrequency energy to the myocardium. The energy is absorbed in the tissue, heating it to a temperature (typically about 50°C) at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

The probe 14 typically comprises a handle 20, having suitable controls on the handle to enable the operator 16 to steer, position and orient the distal tip 18 of the probe 14 as desired for the ablation. To aid the operator 16, a distal portion of the probe 14 contains position sensors 21 that provide signals to a processor 22, located in a console 24. The processor 22 may fulfill several processing functions as described below.

Ablation energy and electrical signals can be conveyed to and from the heart 12 through one or more ablation electrodes 32 located at or near the distal tip 18 via a cable 34 to the console 24. In such a manner, the ablation electrodes 32 are configured to capture electrical potentials over time at multiple sample locations over a surface of one or more chambers of the heart 12. Additionally, or alternatively, other electrodes may be configured to capture electrical potentials over time at multiple sample locations over a surface of one or more chambers of the heart 12. Pacing signals and other control signals may be conveyed from the console 24 through the cable 34 and the electrodes 32 to the heart 12. Sensing electrodes 33, also connected to the console 24, are disposed between the ablation electrodes 32 and have connections to the cable 34. The probe 14 may be implemented without the ablation electrodes 32 as an exploratory device having electrodes 33 configured to capture electrical potentials over time at multiple sample locations over a surface of one or more chambers of the heart 12.

Wire connections 35 link the console 24 with body surface electrodes 30 and other components of a positioning sub-system 27 for measuring location and orientation coordinates of the probe 14. The processor 22 or another processor (not shown) and the position sensors 21, ablation electrodes 32, and sensing electrodes 33 may be elements of the positioning subsystem. The electrodes 32 and the body surface electrodes 30 may be used to measure tissue impedance at the ablation site as taught in U.S. Patent No. 7,536,218. A sensor for bioelectric information, e.g., a temperature sensor (not shown), typically a thermocouple or thermistor, may be mounted on or near each of the electrodes 32.

The console 24 typically contains one or more ablation power generators 25. The probe 14 may be adapted to conduct ablative energy to the heart using any known ablation technique, e.g., radiofrequency energy, ultrasound energy, and laser-produced light energy. Such methods are disclosed in U.S. Patent Nos. 6,814,733, 6,997,924, and 7,156,816.

In one embodiment, the positioning subsystem 27 comprises a magnetic position tracking arrangement that determines the position and orientation of the probe 14 by generating magnetic fields using field generating coils 28 in a predefined working volume and sensing these fields at the probe 14 using the position sensors 21, which are implemented as magnetic coil sensors. The positioning subsystem 27 is described in U.S. PatentNos. 7,756,576, and 7,536,218.

In some embodiments, the processor 22 uses position-signals received from the ablation electrodes 32 and/or the sensing electrodes 33, and the position sensors 21 to estimate a position of the probe 14 inside an organ, such as inside a cardiac chamber. In some embodiments, the processor 22 correlates the position signals received from the electrodes 32, 33 with previously acquired magnetic location-calibrated position signals, to estimate the position of the probe 14 inside the organ. The position coordinates of the electrodes 32, 33 may be determined by the processor 22 based on, among other inputs, measured impedances, or on proportions of currents distribution, between the electrodes 32, 33 and the body surface electrodes 30.

The method of position sensing using current distribution measurements and/or external magnetic fields is implemented in various medical applications, for example, in the Carto® system, produced by Biosense Webster Inc. (Irvine, California), and is described in detail in U.S. Patent Nos. 5,391,199, 6,690,963, 6,484,118, 6,239,724, 6,618,612, 6,332,089, 7,756,576, 7,869,865, and 7,848,787, in PCT Patent Publication WO 96/05768, and in U.S. Patent Application Publication Nos. 2002/0065455 A1, 2003/0120150 A1 and 2004/0068178 A1.

The Carto®3 system applies an Active Current Location (ACL) impedance-based position-tracking method. In some embodiments, using the ACL method, the processor 22 is configured to create a mapping (e.g., current-position matrix (CPM)) between indications of electrical impedance and positions in a magnetic coordinate frame of the field generating coils 28. The processor 22 estimates the positions of the electrodes 32, 33 by performing a lookup in the CPM.

Other methods of determining the location of the distal end of the catheter may be used, for example, based on ultrasonic transducers and receivers, using imaging techniques such as ultrasound or MRI or CT scans which may include disposing radiopaque tags on the probe 14.

As noted above, the probe 14 is coupled to the console 24, which enables the operator 16 to observe and regulate the functions of the probe 14. The processor 22 may be embodied as a computer with appropriate signal processing circuits. The processor 22 is coupled to drive a monitor 29 including a display screen 37. The signal processing circuits typically receive, amplify, filter and digitize signals from the probe 14, including signals generated by sensors such as electrical, temperature and contact force sensors, and the ablation electrodes 32, sensing electrodes 33, position sensors 21 located distally in the probe 14. The digitized signals are received and used by the console 24 to compute the position and orientation of the probe 14, and to analyze the electrical signals from the electrodes 32, 33.

In order to generate electroanatomic maps, the processor 22 typically comprises an electroanatomic map generator, an image registration program, an image or data analysis program and a graphical user interface configured to present graphical information on the monitor 29.

In practice, some or all of these functions of the processor 22 may be combined in a single physical component or, alternatively, implemented using multiple physical components. These physical components may comprise hardwired or programmable devices, or a combination of the two. In some embodiments, at least some of the functions of the processor 22 may be carried out by a programmable processor under the control of suitable software. This software may be downloaded to a device in electronic form, over a network, for example. Alternatively, or additionally, the software may be stored in tangible, non-transitory computer-readable storage media, such as optical, magnetic, or electronic memory.

The console 24 may also include an interface 39 to receive input commands from the operator 16 via any suitable user input device, for example, but not limited to, a pointing device (such as a mouse or stylus), a keyboard, and/or a touch sensitive screen implemented in the display screen 37.

Typically, the system 10 includes other elements, which are not shown in the figures for the sake of simplicity. For example, the system 10 may include an electrocardiogram (ECG) monitor, coupled to receive signals from the body surface electrodes 30, in order to provide an ECG synchronization signal to the console 24. The system 10 may include a reference position sensor, either on an externally applied reference patch attached to the exterior of the subject's body, or on an internally placed probe, which is inserted into the heart 12 maintained in a fixed position relative to the heart 12. Conventional pumps and lines for circulating liquids through the probe 14 for cooling the ablation site may be provided. The system 10 may receive image data from an external imaging modality, such as an MRI unit or the like and includes image processors that can be incorporated in or invoked by the processor 22 for generating and displaying images.

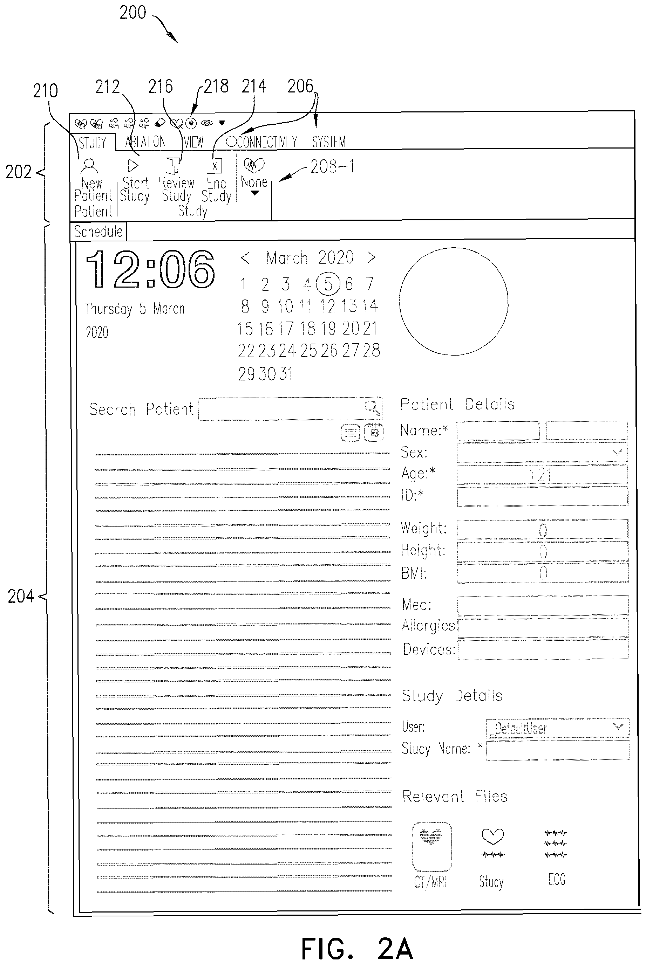

Reference is now made to Figs. 2A-M are views of a user interface screen 200 constructed and operative in accordance with an embodiment of the present invention. The user interface screen 200 includes a tab-based menu 202 above one or more display panes 204. The tab-based menu 202 includes different tabs 206 (only some labeled for the sake of simplicity), which may be selected to reveal a horizontal ribbon 208 associated with the selected tab. The details of the different ribbon 208 are now described in more detail below.

Fig. 2A shows a study ribbon 208-1 as the active ribbon 208 of the user interface screen 200. The study ribbon 208-1 allows the user to perform study related actions. The patient details (currently blank) are shown in a form in the display pane 204 associated with the study ribbon 208-1. Previous patients may be searched and selected in the display pane 204 using a search field. Selecting a new patient action button 210 clears the patient details and allows entry of the new patient details in the form included in the display pane 204. A start study action button 212 commences a new electrophysiological study for the patient whose details are entered in the form. A review study action button 216 commences review of a study selected in the display pane 20subject4. An end study action button 214 ends the currently running study. Fig. 2A also shows a quick-access toolbar 218 above the tab labels. The user may add functions from the tab-based menu 202 to the quick-access toolbar 218 to allow access to the functions (for example, "Add Map", "Delete Point", "Erase FAM") in the quick-access toolbar 218 irrespective of whichever ribbon 208 is currently active. In some embodiments, the quick-access toolbar 218 may be displayed below the active ribbon 208.

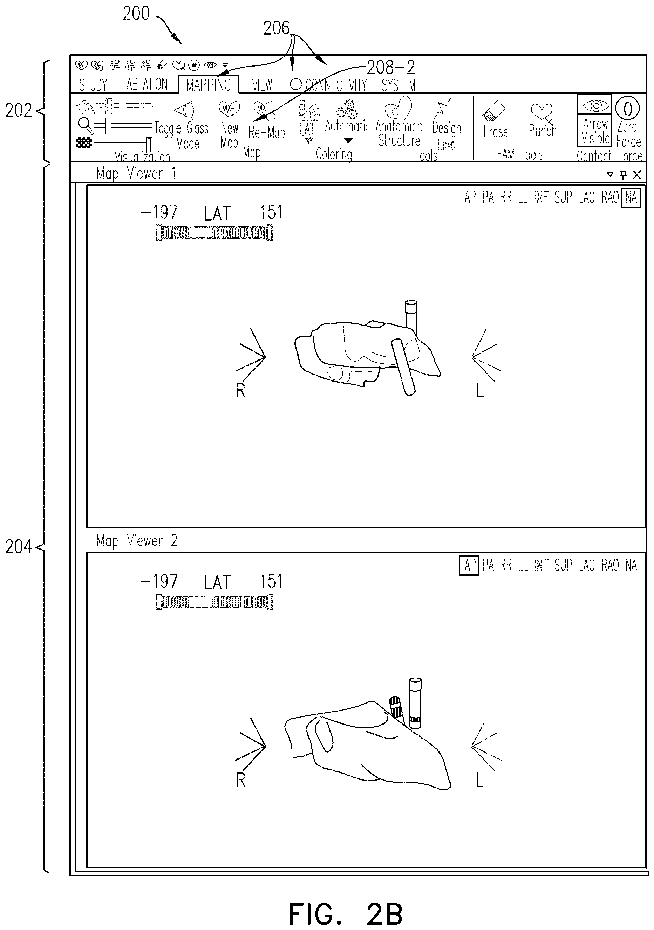

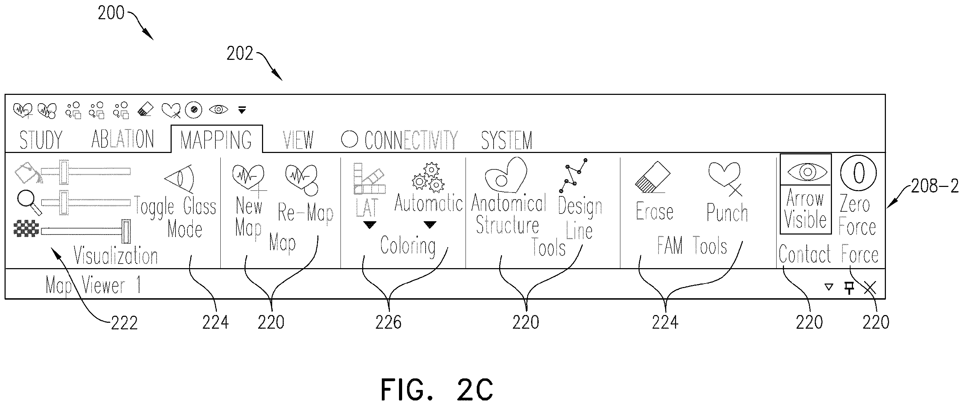

Fig. 2B shows a mapping ribbon 208-2 and example display panes 204 below the mapping ribbon 208-2. The display panes 204 show different map views. Fig. 2C shows an enlarged version of the mapping ribbon 208-2 for clarity. The mapping ribbon tab is generally the default active tab 208 after a new EP study has commenced. The mapping ribbon 208-2 includes various selectable controls (e.g., action buttons 220, sliders 222, toggle buttons 224, drop down buttons 226 - labeled in Fig. 2C) that allow the user to control the visualization settings of the maps shown inside the "Map Viewer" of the display pane 204, and also to allow the user to create new maps by pressing the "New Map" button 220.

The visualization controls are context-aware. In other words, the controls control the selected "Map Viewer" or display pane 204. In Fig. 2B, "Map Viewer 1" is the currently selected display pane 204 so all visualization changes are applied to that display pane 204.

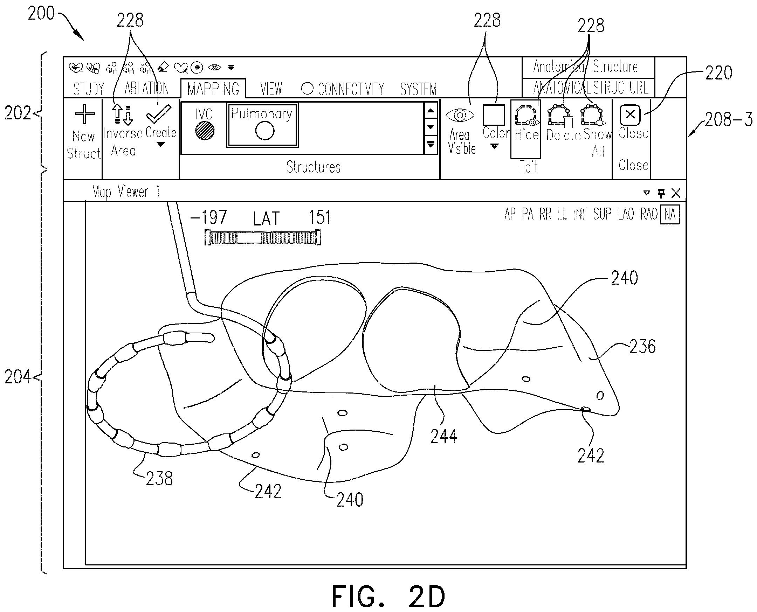

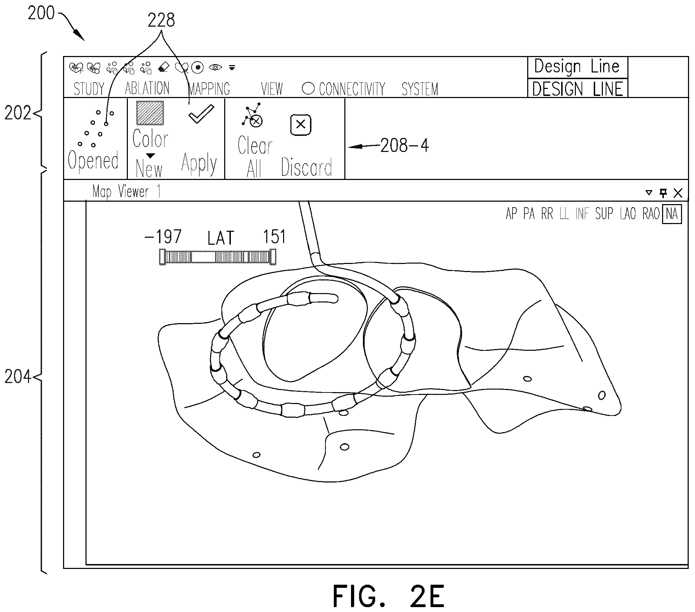

The mapping ribbon 208-2 may control map coloring threshold, window zoom (which controls a zoom value of the selected display pane 204), and map transparency (which controls transparency of the 3D map model of the selected display pane 204) via respective ones of the sliders 222. The "Toggle Glass Mode" toggle button 224 toggles glass mode on and off. The "New Map" button 220 creates a new map. The "ReMap" button 220 restarts mapping. The "LAT" drop down button 226 controls the mapping types, for example, LAT, Unipolar, Bipolar, etc. The "Automatic" drop down button 226 sets the coloring mode, whether the mode should be automatic, manual, or custom. The "Anatomical Structure" button 220 start the anatomical structure creation workflow by showing a contextual tab including an anatomical structure ribbon 208-3 shown in Fig. 2D. The "Design line" buttons 220 starts the design line creation workflow by showing a contextual tab including a design line ribbon 208-4 shown in Fig. 2E. The "Erase" toggle button 224 toggles activation/deactivation of 'Erase FAM' (Fast Anatomical Mapping) mode allowing the user to edit the created 3D volume by erasing a region over which the cursor is dragged. The "Punch" toggle button 224 toggles activation/deactivation of 'Punch FAM' mode allowing the user to edit the created 3D volume by erasing a region, which is defined by the user drawing an outline of the region. The "Arrow Visible" button 220 controls force arrow visibility in the 3D Catheter display in the display pane 204. The "Zero Force" button 220 controls force zeroing.

Fig. 2D shows the anatomical structure ribbon 208-3. The ribbon 208-3 is a part of a contextual tab that appears when the user has pressed the "Anatomical Structure" button 220 in the mapping ribbon 208-2 (Fig. 2C). The ribbon 208-3 includes controls 228 that allow the user to control various aspects of the generated structure such as inverse area, name, color, and area visibility. The ribbon 208-3 is also shown when the user selects a structure in the display pane 204, and allows the user to change existing structure properties as well as to show/hide or delete the structure. The 'Close' button 220 closes the tab of the ribbon 208-3.

Fig. 2D also shows an anatomical map 236, a representation 238 of a probe, an anatomical structure 244, electrophysiological data indicators 240 (e.g., LAT coloring) (only some labeled for the sake of simplicity), and ablation indicators 242 (only some labeled for the sake of simplicity) in the display pane 204 of the user interface screen 200.

Fig. 2E shows the design line ribbon 208-4. The design line ribbon 208-4 is part of a contextual tab that appears when a user chooses to add a new design line from the mapping ribbon 208-2 of Fig. 2C. The ribbon 208-4 includes controls 228 that control the color and opened/closed state of the generated design line.

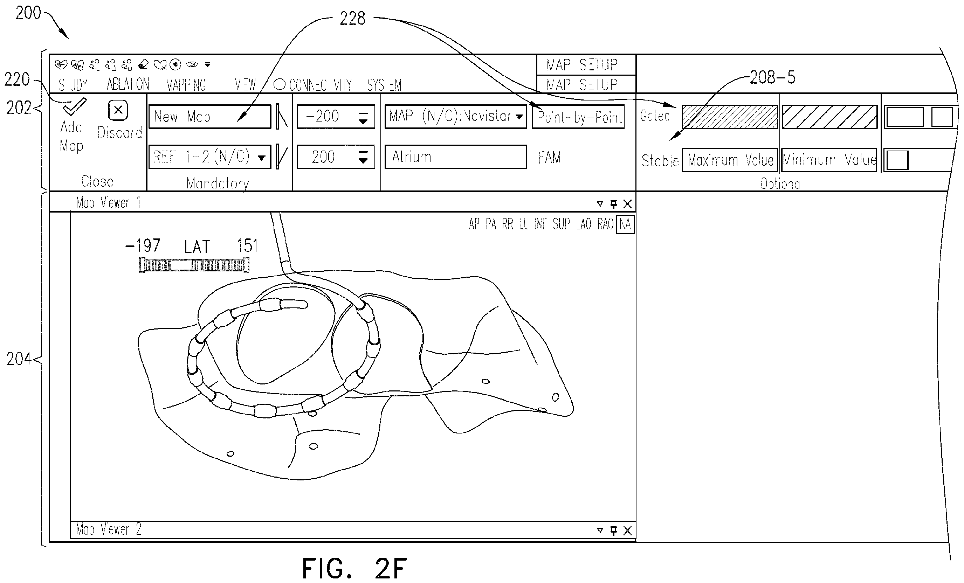

Fig. 2F shows a map setup ribbon 208-5, which is part of a contextual tab which is activated when the user chooses to add a new map from the mapping ribbon 208-2. The ribbon 208-5 includes controls 228 that allow the user to set map properties including name, reference channel, window of interest (WOI), type etc. The ribbon 208-5 also includes color pickers that allow the user to set the colors of the reference and mapping annotations. An 'Add Map' button 220 is a confirmation button, which closes the tab and adds the created map to the 3D Window of the display pane 204.

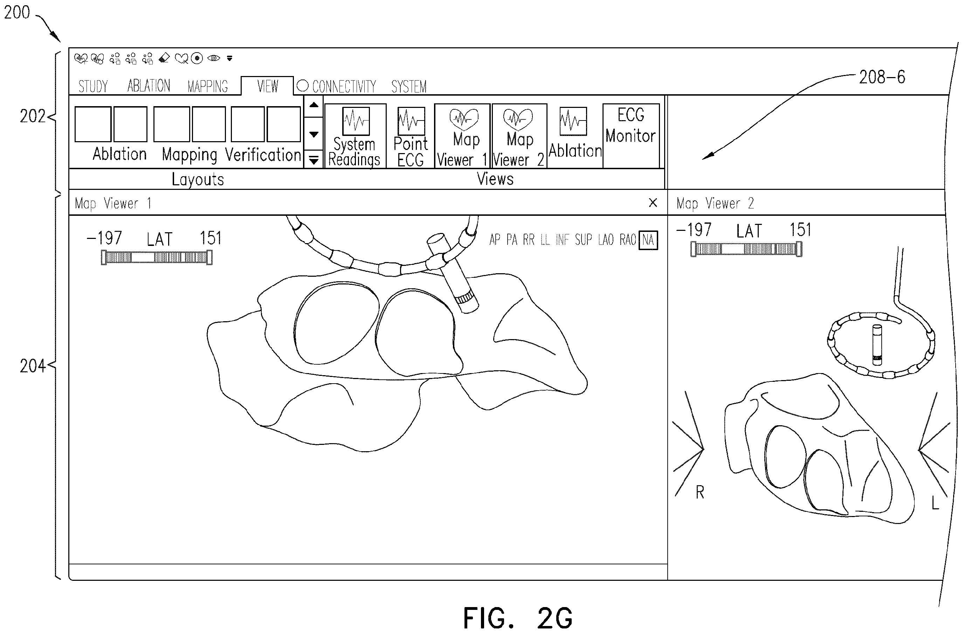

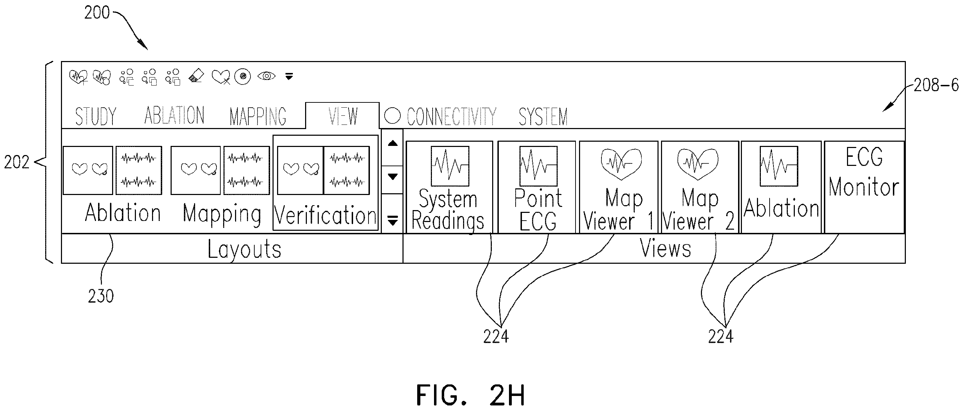

Fig. 2G shows a view ribbon 208-6 and example display panes 204 below the view ribbon 208-6. The display panes 204 show different map views. Fig. 2H shows an enlarged version of the view ribbon 208-2 for clarity. The ribbon 208-6 includes a gallery 230 (Fig. 2H) of available screen layouts. Selecting a layout changes the layout of views in the display panes 204. The ribbon 208-6 also includes visibility toggle buttons 224 (Fig. 2H) for each available view shown in the display panes 204 allowing views to be selected and deselected for showing in the display panes 204.

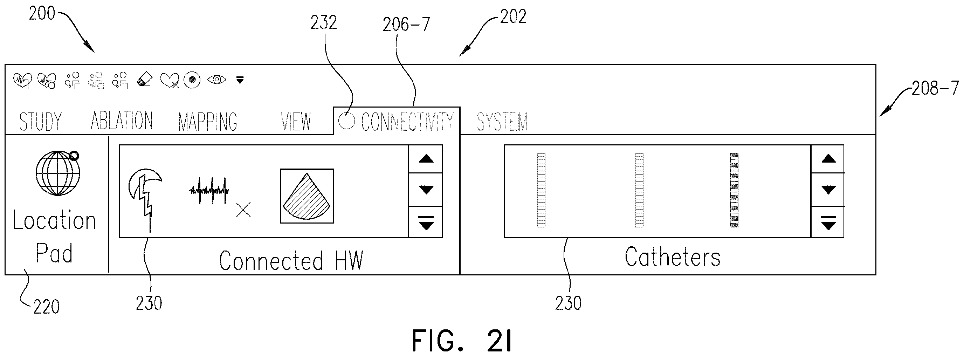

Fig. 2I shows a connectivity ribbon 208-7. The ribbon 208-7 includes two galleries 230 one for connection hardware (which includes the hardware elements connected to the mapping system 10 and the state of the hardware elements), and one for catheters and probes (which includes a list of the connected catheters/probes). The ribbon 208-7 includes a 'Location Pad' button 220 which when selected shows a location pad setup screen (not shown). An indicator 232 is included on the title of the connectivity tab 206-7, and indicates the aggregate connectivity status of the hardware and catheters to allow the user quick access to the status even when the tab 206-7 is not selected. For example, the indicator 232 may be green when all the hardware and catheters are connected, otherwise the indicator 232 is another color, e.g., red or gray.

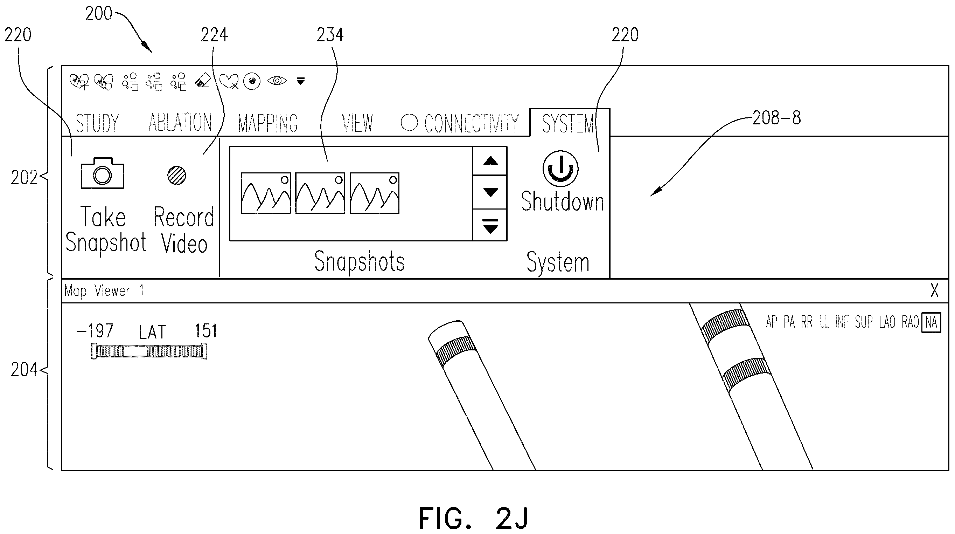

Fig. 2J shows a system ribbon 208-8, which includes various controls including: a "Take Snapshot" button 220 which creates a screen snapshot of the display pane 204 (only partially shown in Fig. 2J) and adds the snapshot to a snapshot gallery 234; a "Record Video" toggle button 224 which toggles recording of the display pane 204; and a shutdown button 220, which shuts down the software application of the mapping system 10. Recording may also be added to the snapshot gallery 234.

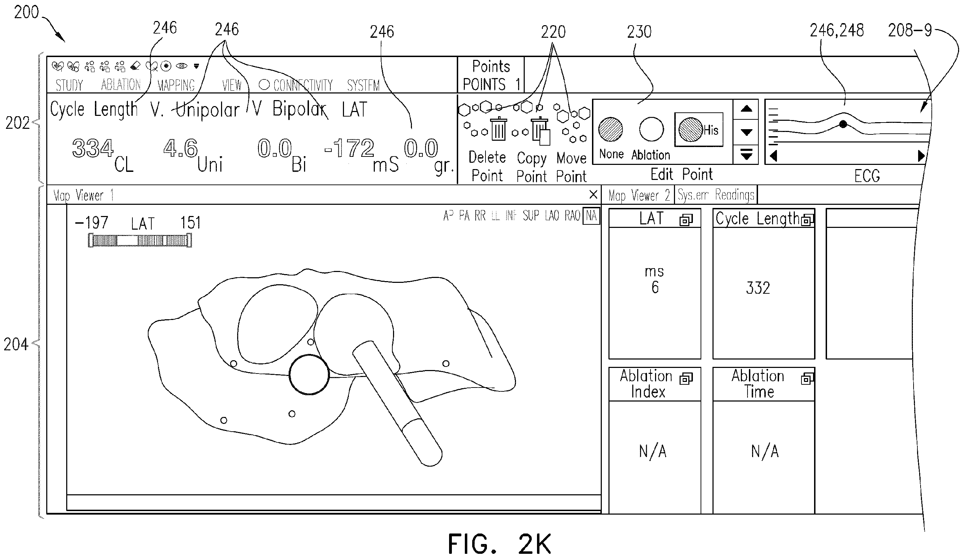

Fig. 2K shows a points ribbon 208-9, which is part of a contextual tab including both information, in the form of data readout elements 246, and user interaction controls. The points ribbon 208-9 is generally shown when the user selects a point in the display pane 204 or via a points list. The data readout elements 246 include readings about the selected point such as voltage, LAT, cycle length and other characteristics of the selected point, and an ECG 248, which displays the reference and mapping channel ECG data around the selected point and marks the mapping and reference annotations on the ECG. The points ribbon 208-9 also includes user action buttons 220 including "Delete Point", "Copy Point", "Move Point", and a tag gallery 230 which allows selecting a tag from the tag gallery 230 to apply the selected tag to the selected point.

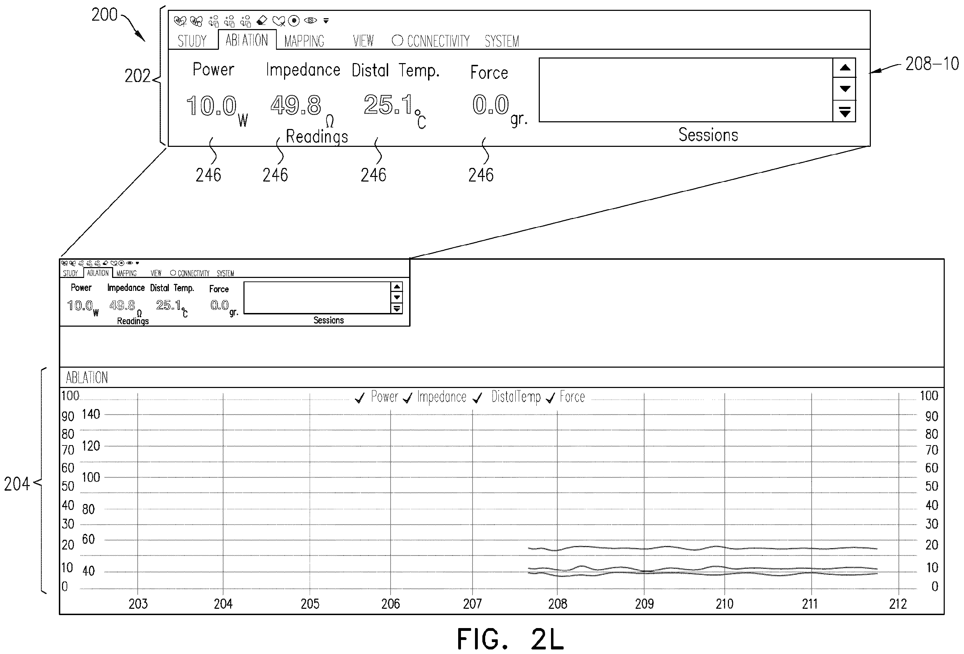

Fig. 2L shows an ablation ribbon 208-10 including data readout elements 246 displaying real-time ablation readings of a currently occurring ablation such as power, impedance (of tissue), temperature (of tissue or of the distal end of the probe 14 (Fig. 1) and a force applied by the probe 14 on the tissue, by way of example. The display pane 204 may include a graphical representation of the ablation readings over time.

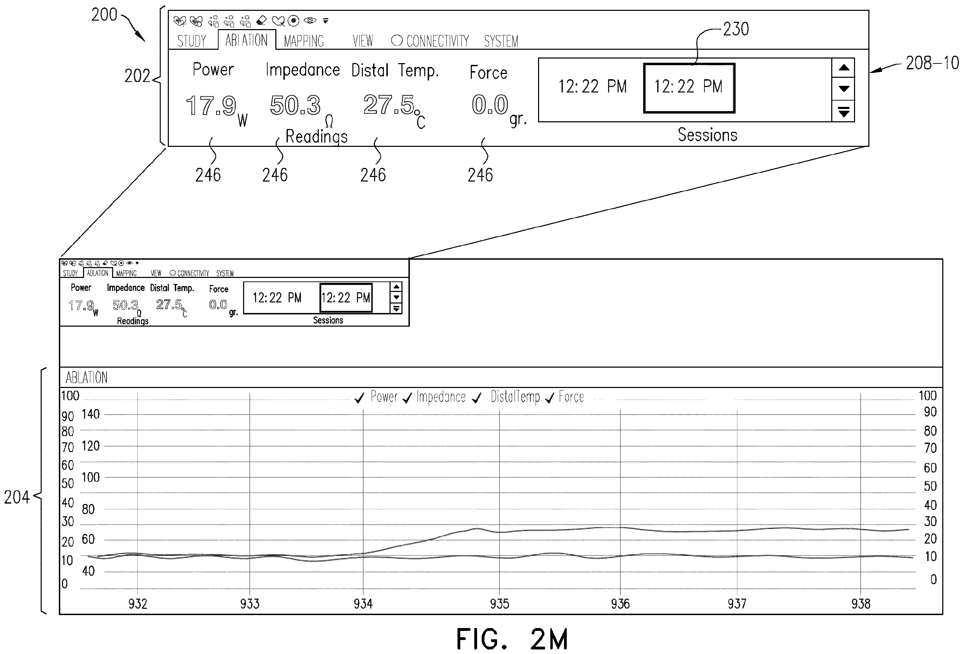

Fig. 2M shows ablation ribbon 208-10 when ablation is not currently being performed. The ablation ribbon 208-10 includes an ablation sessions gallery 230, which lists the various ablation sessions that have been previously performed (for example, by time and/or date). Selecting one of the previous sessions from the gallery 230 displays the respective ablation data in the data readout elements 246 and in an ablation data graph displayed in the display pane 204. The data readout elements 246 include data of average accumulated ablation values for power, impedance, temperature and force, during the selected ablation session.

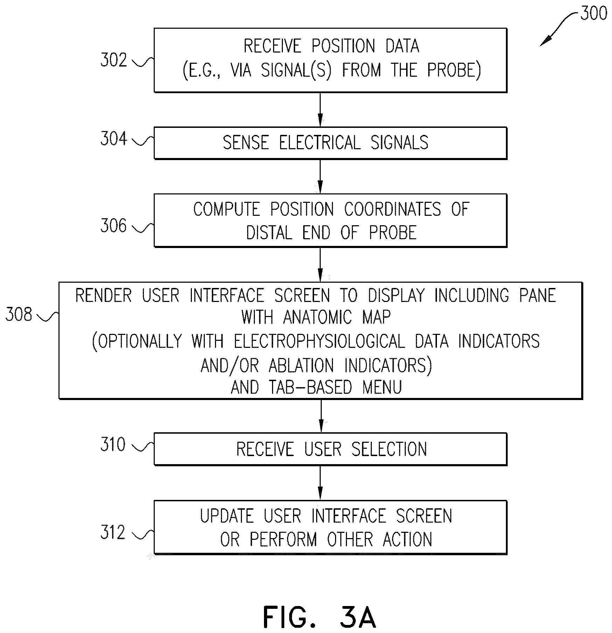

Reference is now made to Fig. 3A, which is flowchart 300 including steps in a method of operation of the mapping system 10 of Fig. 1. Reference is also made to Fig. 1.

The processor 22 may be configured to receive (block 302) position data from the positioning sub-system 27, which is configured to sense a position of the probe 14 inserted into the body cavity of a living subject 38. The position data may be received by the processor 22 in at least one signal from one or more of the position sensors 21 and/or one or more of the electrodes 32, 33, or from the body surface electrodes 30 or from any other suitable sensor depending on the position sensing method being implemented. The probe 14 is an example of a probe or catheter which may be inserted into the body cavity. Other probes or catheters may be used, for example, a multi-electrode catheter including flexible splines, a lasso catheter, a basket catheter, or a balloon catheter.

The processor 22 may be configured to sense (block 304) electrical signals (e.g., electrical activation signals) of the body cavity with one or more of the electrodes 32, 33 of the probe 14, or any other suitable probe. The electrical signals may be used by the processor 22 to enhance an anatomical map with electrical activity indicators, for example, using LAT shading or coloring or vectors. The processor 22 may be configured to compute (block 306) position coordinates of the distal end of the probe 14 responsively to the position data.

The processor 22 is configured to render (block 308), to the display screen 37, the user interface screen 200 (Figs. 2A-M) including: the display pane(s) 204 including the anatomical map 236 (Fig. 2D) of the body cavity responsively to the computed position coordinates; the tab-based menu 202 (Figs. 2A-M) above the pane(s) 204. In some embodiments, the processor 22 is configured to render the anatomical map 236 responsively to the signal(s) received from any one or more of the following: the positioning sub-system 27, one or more of the position sensors 21, one or more of the electrodes 32, 33, the body surface electrodes 30, any other suitable sensor. The signal(s) may be used to generate the anatomical map 236 using any suitable mapping method based on position signals and/or the sensed electrical activity. The processor 22 may be configured to render the anatomical map 236 with electrophysiological data indicators 240 (Fig. 2D) (e.g. a LAT map using coloring, shading and/or vectors) responsively to the sensed electrical signals. The processor 22 may also be configured to render the representation 238 (Fig. 2D) of the probe 14 in the display pane(s) 204 responsively to the computed position coordinates of the distal end of the probe 14. In some embodiments, the processor 22 may be configured to render ablation indicators 242 (Fig. 2D) (e.g., VisiTags) on the anatomical map 236. The display pane(s) 204 may include one or more representations of the probe(s) that move in the pane(s) according to the computed position(s) of the distal end(s) of the probe(s). The display pane(s) 204 may include one or more anatomical maps 236 and one or more views of each anatomical map 236.

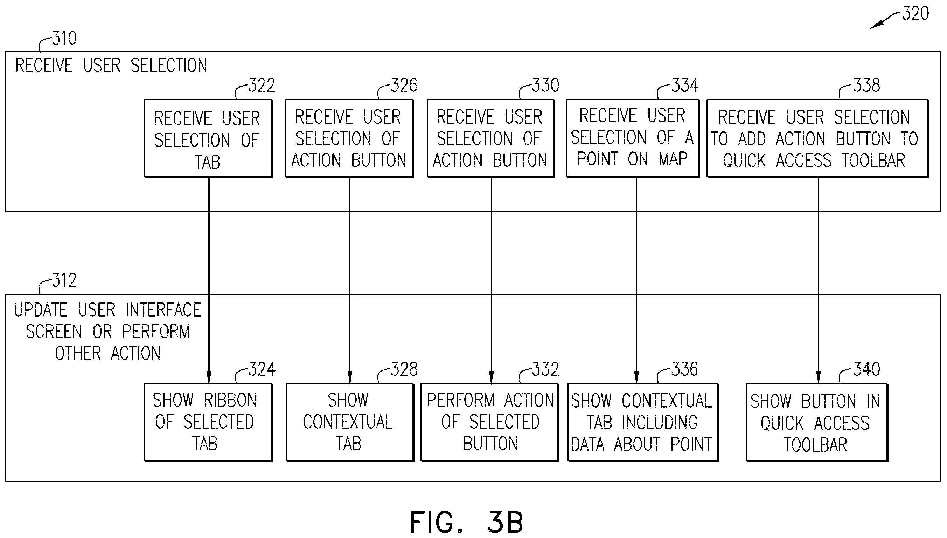

The processor 22 is configured to receive (block 310) a user selection from the tab-based menu 202 and update (block 312) the user interface screen 200, or perform another action (e.g., configure the probe 14 or any suitable probe, catheter or peripheral hardware connected to the mapping system 10). The steps of blocks 310 and 312 are described in more detail with reference to Fig. 3B.

The steps described above may be performed in any suitable order and may be repeated intermittently. For example, while the user interface is being displayed and user selections are being received and processed, signals may be received from the probe 14 (Fig. 1) and/or the positioning sub-system 27 (Fig. 1) for processing as described above.

Reference is now made to Fig. 3B, which is a flowchart 320 including sub-steps of some of the steps of the flowchart 300 of Fig. 3A. Reference is also made to Fig. 1. Sub-steps of blocks 310 and 312 are now described below.

The processor 22 may be configured to receive (block 322) a user selection selecting the tab 206 of the tab-based menu 202, and show (block 324) one of the horizontal ribbons 208 (Figs. 2A-M) of the selected tab above the pane(s) 204 in the user interface screen 200 responsively to the received user selection. The displayed ribbon 208 is also referred to as the "active ribbon" below. The display pane(s) 204 is (are) generally not resized, or even partially hidden by the active ribbon 208, responsively to showing the horizontal ribbon 208 in the tab-based menu 202.

Each ribbon 208 may include one or more data readout elements about any one or more of the following: ablation data (Figs. 2L and 2M); a tissue property (such as impedance in Figs. 2L and 2M); a selected point of the anatomical map (see Fig. 2K); and tool connectivity (see Fig. 2I). Additionally, or alternatively, the ribbon 208 may include one or more controls such as action buttons 220 or sliders 222 or toggles 224 or pull down button 226 (see Fig. 2C) to perform any one or more of the following: creation of the anatomical map 236 (see Fig. 2C and 2F); formatting the anatomical map 236 (see Fig. 2C); creation of the anatomical structure 244 (Fig.2D); editing the anatomical map 236; configuring the probe 14, taking a screen snapshot of the pane(s) 204 (Fig. 2J); recording the pane(s) 204 (Fig. 2J); selecting a preset layout of which pane(s) to show; and creating custom layouts (see Fig. 2H).

The processor 22 may be configured to receive (block 326) a user selection of a selectable control of the active ribbon 208, and show (block 328) a contextual tab 206 in the user interface screen 200 responsively to the user selection of the selectable control. A contextual tab 206 is a tab which is not shown in the tab-based menu unless it is activated based on performing a certain action (e.g., such as selecting a point of the anatomical map 236, as described in more detail below and with reference to Fig. 2K) or based on selecting a certain control (e.g., a create new map button 220 (Fig. 2C) or a create anatomical structure button 220 (Fig. 2C)). Once activated, the contextual tab 206 may include readout elements 246 (Fig. 2K) and/or selectable controls. The contextual tab 206 generally remains open until a particular action or set of actions has been completed (e.g., a new map has been created or an anatomical structure has been created) or the contextual tab 206 is closed by a user.

The processor 22 may be configured to receive (block 330) a user selection of a selectable control of the active ribbon 208, and perform (block 332) an action associated with the selected control.

The processor 22 may be configured to receive (block 334) a user selection of a point on the anatomical map 236 (for example, by clicking, double clicking, or hovering over a point or mark or tag (e.g., VisiTag)), and show (block 336) a contextual tab 206 in the user interface screen 200 responsively to the user selection of the point. The contextual tab 206 may include data about the selected point and/or context relevant controls.

The processor 22 may be configured to receive (block 338) a user selection of a selectable control of the active ribbon 208 (or from a list of controls) to add the selectable control to the quick-access toolbar 218 (Fig. 2A), and show (block 340) a miniaturized representation of the selectable control in the quick access toolbar 218.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 72% to 108%.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

## Claims

1. A medical mapping method comprising:
receiving position data from a positioning sub-system configured to sense a position of a probe inserted into a body cavity of a living subject;
computing position coordinates of a distal end of the probe responsively to the position data;
rendering to a display a user interface screen including:
a display pane including an anatomical map of the body cavity responsively to the computed position coordinates; and
a tab-based menu above the pane;
receiving a user selection selecting a tab of the tab-based menu; and
showing a horizontal ribbon of the selected tab above the pane in the user interface screen responsively to the received user selection.

2. The method according to claim 1, further comprising:
receiving a user selection of a selectable control of the ribbon; and
showing a contextual tab in the user interface screen responsively to the user selection of the selectable control, the contextual tab including other selectable controls.

3. The method according to claim 1, further comprising:
receiving a user selection of a point on the anatomical map; and
showing a contextual tab in the user interface screen responsively to the user selection of the point, the contextual tab including data about the selected point.

4. The method according to claim 1, further comprising:
receiving a user selection of a selectable control of the ribbon to add the selectable control to a quick access toolbar; and
showing a miniaturized representation of the selectable control in the quick access toolbar.

5. The method according to claim 1, further comprising:
receiving at least one signal from at least one sensor of the probe; and
rendering the anatomical map responsively to the at least one signal.

6. The method according to claim 5, further comprising sensing electrical signals of the body cavity with at least one electrode of the probe, wherein the rendering the anatomical map includes rendering the anatomical map with electrophysiological data indicators responsively to the sensed electrical signals.

7. The method according to claim 1, further comprising rendering a representation of the probe in the display pane responsively to the computed position coordinates, rendering ablation indicators on the anatomical map.

8. A medical system, comprising: a probe including a distal end, and configured to be inserted into a body cavity of a living subject; a positioning sub-system configured to sense a position of the probe; a display; and a processor configured to:
receive position data from the positioning sub-system;
compute position coordinates of the distal end of the probe responsively to the position data;
render to the display a user interface screen including:
a display pane including an anatomical map of the body cavity responsively to the computed position coordinates; and
a tab-based menu above the pane;
receive a user selection selecting a tab of the tab-based menu; and
show a ribbon of the selected tab in the user interface screen responsively to the received user selection.

9. The method according to claim 1 or the system according to claim 8, wherein the display pane is not resized or partially hidden responsively to the showing of the horizontal ribbon.

10. The system according to claim 8, wherein the processor is configured to:
receive a user selection of a selectable control of the ribbon; and
show a contextual tab in the user interface screen responsively to the user selection of the selectable control, the contextual tab including other selectable controls.

11. The method according to claim 2 or the system according to claim 10, wherein the other selectable controls of the contextual tab include controls to create a new map or a new anatomical structure from an existing map.

12. The system according to claim 8, wherein the processor is configured to:
receive a user selection of a point on the anatomical map; and
show a contextual tab in the user interface screen responsively to the user selection of the point, the contextual tab including data about the selected point.

13. The method according to claim 1 or the system according to claim 8, wherein the ribbon includes at least one data readout element about any one or more of the following: ablation data; a tissue property; a selected point of the anatomical map; and tool connectivity.

14. The method according to claim 1 or the system according to claim 8, wherein the ribbon includes at least one control to perform any one or more of the following: creation of the anatomical map; formatting the anatomical map; creation of an anatomical structure; editing the anatomical map; configuring the probe, taking a screen snapshot of the pane; recording the pane; selecting a preset layout of which panes to show; and creating custom layouts.

15. The system according to claim 8, wherein the processor is configured to:
receive a user selection of a selectable control of the ribbon to add the selectable control to a quick access toolbar; and
show a miniaturized representation of the selectable control in the quick access toolbar.

16. The system according to claim 8, wherein:
the probe includes at least one sensor; and
the processor is configured to:
receive at least one signal from the at least one sensor of the probe; and
render the anatomical map responsively to the at least one signal.

17. The system according to claim 16, wherein:
the probe includes at least one electrode, which is configured to sense electrical signals of the body cavity; and
the processor is configured to render the anatomical map with electrophysiological data indicators responsively to the sensed electrical signals.

18. The system according to claim 8, wherein the processor is configured to render a representation of the probe in the display pane responsively to the computed position coordinates, or to render ablation indicators on the anatomical map.

19. A software product, comprising a non-transient computer-readable medium in which program instructions are stored, which instructions, when read by a central processing unit (CPU), cause the CPU to:
receive position data from a positioning sub-system configured to sense a position of a probe inserted into a body cavity of a living subject;
compute position coordinates of a distal end of the probe responsively to the position data;
render to a display a user interface screen including:
a display pane including an anatomical map of the body cavity responsively to the computed position coordinates; and
a tab-based menu above the pane;
receive a user selection selecting a tab of the tab-based menu; and
show a ribbon of the selected tab in the user interface screen responsively to the received user selection.
